# EUROPEAN PATENT SPECIFICATION

(11) **EP 2 749 276 B1**
(45) Date of publication and mention of the grant of the patent: **02.08.2017**
(21) Application number: 11871448.4
(22) Date of filing: 22.10.2011
(51) Int. Cl.: A61K 31/045, A61K 9/00, A61P 1/04

(54) **USE OF PATCHOULI ALCOHOL IN PREPARATION OF DRUG AGAINST HELICOBACTER PYLORI**
VERWENDUNG VON PATSCHULIALKOHOL BEI DER HERSTELLUNG EINES ARZNEIMITTELS GEGEN HELICOBACTER PYLORI
UTILISATION DE L'ALCOOL DE PATCHOULI DANS LA PRÉPARATION D'UN MÉDICAMENT CONTRE HELICOBACTER PYLORI

(30) Priority: 26.08.2011 CN 201110249038
(43) Date of publication of application: 02.07.2014
(73) Proprietor: Dongguan Mathematical Engineering Academy of Chinese Medicine Guangzhou University of Chinese Medicine, Dongguan, Guangdong 523808 (CN)
(72) Inventor: LAI, Xiaoping, Dongguan Guangdong 523808 (CN); SU, Ziren, Dongguan Guangdong 523808 (CN); CHEN, Jiannan, Dongguan Guangdong 523808 (CN); LI, Yucui, Dongguan Guangdong 523808 (CN); HE, Jingjin, Dongguan Guangdong 523808 (CN)
(74) Representative: Gulde & Partner
(86) International application number: PCT/CN2011/081153
(87) International publication number: WO 2013/029297

(56) References cited:
- CN-A- 1 772 017
- CN-A- 1 822 767
- CN-A- 101 194 899
- CN-A- 101 485 647
- CN-A- 101 485 647
- JP-A- H07 196 522
- US-A1- 2006 134 239
- XIAN YAN-FANG ET AL: "Anti-inflammatory effect of patchouli alcohol isolated from Pogostemonis Herba in LPS-stimulated RAW264.7 macrophages", EXPERIMENTAL AND THERAPEUTIC MEDICINE, vol. 2, no. 3, May 2011 (2011-05), pages 545-550, XP002736696,
- FENNERTY B ET AL: "HELICOBACTER PYLORI: REVIEW OF TRIPLE, DUAL, 7-DAY, AND OTHER TREATMENT STRATEGIES", FORMULARY, ADVANSTAR COMMUNICATIONS, CLEVELAND, OH, US, vol. 30, no. 11, November 1995 (1995-11), pages 682-688, XP000670115, ISSN: 1082-801X

## Description

### Background of the Present Invention

### Description of Related Arts

Patchouli alcohol (Guang Huo Xiang Chun in Chinese pinyin) is also known as Bai Qiu Li Chun and Hu Wei Cao Chun (in Chinese pinyin). Patchouli alcohol is a tricyclic sesquiterpene which has a chemical name of (1R,4S,4aS,6R,8aS)-4,a,9,9-tetramethyloctahydro-1,6-methanonaphthalen-1(2H)-ol, a molecular formula of C₁₅H₂₆O, a molar mass of 222.37, and a structural formula which is illustrated in formula (I) below. Its appearance is a hexagonal-trapezohedral crystal, and it is insoluble in water, soluble in ethanol and ether and common organic solvent. The European patent application, publication number WO2004110153A1, discloses patchouli alcohol and its derivatives for inhibiting the asexual propagation of fungi, for reducing the adhesion effect of microorganisms to surfaces, and its effect on wound-cleaning and wound-healing. The

Chinese patent application, publication number CN101194899A, discloses the use of patchouli alcohol in preparation of pharmaceutical composition for preventing and treating Alzheimer's Disease. The Chinese patent application, publication number CN101485647A, discloses the significant effect of patchouli alcohol in inhibiting and killing influenza A1 virus, influenza B virus and avian influenza virus H5N1.

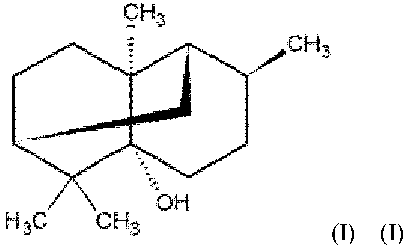

Patchouli alcohol has a very complex chemical structure and its synthesis is complex and lengthy. Therefore, the major source of patchouli alcohol comes from patchouli oil. Patchouli oil is the oil extract from *Pogostemon cablin* (Blanco) Benth. which belongs to the labiatae family. Patchouli oil has stimulating effect on gastric secretion and therefore enhances digestion. Patchouli oil also has antispasmodic effect and anti-bacterial and anti-Trichophyton effect on skin.

In JP H 07196522 A anti-Helicobacter pylori activators are disclosed for preventing or treating gastritis and gastric and duodenal ulcer.

### Summary of the Present Invention

The present invention is directed to subject matter as defined in the claims.

An object of the present invention is to provide patchouli alcohol for a new use, which show a new use in pharmaceutical preparation.

The new use refers to the application of Patchouli Alcohol in pharmaceutical preparation of anti-*Helicobacter pylori* medication.

The patchouli alcohol has a complex chemical structure and mature synthetic method of patchouli alcohol is not common. At present, patchouli alcohol is obtained by first extracting patchouli oil from patchouli and then separating patchouli alcohol from the patchouli oil. The Chinese patent application, publication number CN10148647A, discloses the details of the separation method.

The anti-*Helicobacter pylori* medication for use according to the present invention comprises patchouli alcohol and pharmaceutically acceptable additives, wherein the content of patchouli alcohol is 0.5∼70% (by percentage weight). The medication can have a plurality of application forms which include injection form or other common application forms such as oral form, which includes tablet, soft capsule and dripping pill.

*Helicobacter pylori,* abbreviation Hp, is classified as Campylobacterales and belongs to Helicobacteraceae family. Hp is a gram-negative bacterium which has multiple unipolar flagella, rounded end and spiral shape. Since *Helicobacter pylori* can easily develop drug resistance properties, treatment regimen which only utilizes antibiotics or antibacterial agents is rare. The common treatment is triple therapy which includes a proton pump inhibitor or bismuth and an additional two antibiotics selected from clarithromycin, amoxicillin and metronidazole. All of the antibiotics used in the triple therapy belong to broad spectrum antibiotics and periodic or overdose usage will cause two major problems. First, the bacteria will develop drug resistance property, and second, the probiotics will also be killed. A long-term research of the present invention reveals that the antibacterial spectrum of patchouli alcohol is very narrow, and patchouli alcohol can have selective inhibitory effect on *Helicobacter pylori* without affecting the growth and reproduction of other gram-negative bacteria. Patchouli alcohol specifically kills *Helicobacter pylori* without imposing any harmful effect on other probiotics, therefore the ecological balance of the intestinal flora in human can be maintained.

Advantages and features of patchouli alcohol for use according to the present invention in inhibiting *Helicobacter pylori* are demonstrated through pharmacological testing in the followings.

### I. In vitro Experiment on anti-Helicobacter pylori effect

### 1. Test samples:

Patchouli alcohol: Prepare patchouli alcohol by using the method of preparation according to the disclosure of the Chinese patent application, publication number CN1014856471A, to obtain patchouli alcohol which has a purity greater than 98%; obtain the patchouli alcohol and polyoxyethylene hydrogenated castor oil (RH40), which is 8 times by weight, and add a suitable amount of purified water to obtain a patchouli alcohol solution with a concentration of 5mg/ml.
Proton pump inhibitor, PPI: Lansoprazole, bought from China National Institutes for Food and Drug Control, batch number: 100709-200501.
Clarithromycin: obtained from China National Institutes for Food and Drug Control, batch number: 130356-200403.
Amoxicillin: Product of Zhuhai United Laboratories Inc., batch number: 00800208.
Culture medium: Columbia Blood Agar Plate
Testing Bacteria: *Helicobacter pylori* (Hp), obtained from American Type Culture Collection, Strain number: ATCC43504, LOT: 3965862.

### 4. Testing method

### 4.1: Drug preparation and dilution

Take 13 sterile tubes (8 × 100mm) and arrange in a row, add 1ml Brucella broth to each of the tubes, then add 1.0ml patchouli alcohol into tube 1 and mix uniformly, draw 1ml solution from tube 1 and add it to tube 2 and mix uniformly, then draw 1ml solution from tube 2 and add it to tube 3 and mix uniformly, continue the above steps for the remaining tubes until 1ml solution from tube 10 is added to tube 11 and mix uniformly, then draw and discard 1ml solution from tube 11. Tube 12 is for growth control and no patchouli alcohol is added. Accordingly, the drug concentration of the tube 1 to the tube 12 are 0.5, 0.25, 0.125, 0.0625, 0.0312, 0.0156, 0.0078, 0.0039, 0.0019, 0.0010 and 0.0005ml/ml respectively. Then add 0.1ml inoculum prepared from the above method into each tube to obtain the final bacterial concentration at approximately 2.48×10⁸CFU/ml.

Cultivation of Bacteria: add 100µl 2.48×10⁹CFU/ml bacterial solution to Columbia Blood Agar Plate, spread uniformly, soak perforated filter paper (2mm in diameter) into the above diluted solution and place onto the Columbia blood agar plate in which the bacterial solution is added. Place 3 pieces for each plate and 3 plates for each concentration.

Incubation: Place in 37°C micro-aerobic culture bag and incubate in an incubation shaker for 72 hours.

### Results Determination

Determine the minimum inhibitory concentration. MIC is the lowest concentration of an antimicrobial that will inhibit the visible growth of a microorganism. After incubation, a circular inhibition zone can be formed around the test strip. The test results show that the MIC of patchouli alcohol is 0.000078g/ml, the MIC of Triple therapy (lansoprazole, clarithromycin, and amoxicillin, at a ratio of 0.2:5:10) is 0.000096g/ml; and the MIC of Amoxicillin is 0.00012 g/ml. The test results show that patchouli alcohol has inhibitory effect on *Helicobacter pylori* and the effect is greater than the Triple therapy treatment regimen, which serves as the positive control.

### II. In vivo Experiment on anti-Helicobacter pylori effect

Test samples are identical to the samples of the above *in vitro* experiment on anti-*Helicobacter pylori* effect.

Take SPF (specific pathogen free) BALB/c mice, equal numbers of male and female, each weight 18∼20g; divide randomly into three groups, which are normal control group, model control group and triple therapy group. Give the corresponding experimental drugs according to table 1 below. Hp infection model is prepared by using antibiotics and oral gavage of Hp. Day 1, oral gavage 0.5ml/mouse antibiotics mixture (0.25ml ampicillin (20mg/ml), 0.15ml gentamicin (4mg/ml), 0.1ml azithromycin (50mg/ml)) for three consecutive days for all testing animals, use equal volume of physiological saline for normal control group; starting from day 4, water fasting for 12 hours, oral gavage freshly prepared Hp suspended solution (0.5ml/mouse/day, concentration 10⁸ CFU/ml) for seven consecutive days, use equal volume of physiological saline for normal control group. Starting from day 11, oral gavage testing drug to the testing animals (use equal volume of physiological saline for normal control group), 1 time per day for 8 consecutive weeks. After the last drug administration, fasting for 24 hours. Take serum sample by orbital bleeding and centrifugation. Test for *Helicobacter pylori* urease antibody (according to the instruction of the instrument); sacrifice mice, tissue from pyloric antrum is first taken for rapid urease test and then observe for 3 minutes, and determine the test results in which strong positive refers to color change at peripheral portion from yellow to red within 1 minute, weak positive refers to color change within 3 minutes, and negative refers to no color change; the remaining stomach tissue is fixed in 10% formalin, then embed and cutting for carbol-fuchsin test, observe the Hp infection condition of the gastric mucosa and determine the condition. The results are shown in Table 1 and Table 2.

**Table 1: Inhibitory effect on Helicobacter pylori of Patchouli Alcohol (Rapid Urease Test)**

| Group | Number of Animal (count) | Dosage (mg/kg) | Strong positive (count) | Weak positive (count) | Total Positive Rate (%) |
|---|---|---|---|---|---|
| Normal Control | 10 | - | 0 | 0 | 0.00 |
| Model Control | 11 | - | 1 | 7 | 72.73^{▲▲} |
| Triple therapy | 11 | 670 | 1 | 3 | 36.36 |
| Patchouli Alcohol Low Dose | 11 | 10 | 0 | 4 | 36.36 |
| Patchouli Alcohol Medium Dose | 11 | 20 | 0 | 3 | 27.27* |
| Patchouli Alcohol High Dose | 10 | 40 | 2 | 2 | 40.00 |

| | | | | | |
|---|---|---|---|---|---|
| Remarks: Compare to normal control group, ^{Δ}P<0.05, ^{ΔΔ}P<0.01 (Same below). Compare to model control group, *P<0.05, **P<0.01 (Same below). | | | | | |

The results in Table 1 show that the total positive rate of Hp infection between the model group and the normal group has a significant difference, which means that the model is built successfully. Compare to model control group, the Patchouli Alcohol Medium Dose group has a significant lower total positive rate (p<0.05).

**Table 2: Inhibitory effect on Helicobacter pylori of Patchouli Alcohol (Serum Urease antibody to Helicobacter pylori)**

| Group | Dosage (mg/kg) | Number of Animal (count) | Positive (Count) | Positive rate (%) |
|---|---|---|---|---|
| Normal Control | - | 10 | 0 | 0.00 |
| Model Control | - | 11 | 9 | 81.82^{▲▲} |
| Triple Therapy | 670 | 11 | 0 | 0.00** |
| Patchouli Alcohol | 10 | 11 | 0 | 0.00** |
| Patchouli Alcohol | 20 | 11 | 0 | 0.00** |
| Patchouli Alcohol | 40 | 10 | 0 | 0.00** |

The results in Table 2 show that the total positive rate of Hp infection of the model group, as compared to normal group, is increased significantly (p<0.01), which means that the model is built successfully. Compare to model control group, the Patchouli Alcohol Low, Medium and High Dose groups have a significant lower total positive rate (p<0.01).

Histopathological findings: after staining stomach tissue by carbol-fuchsin method, the Hp become blue in color, have a curved or rod shape, and mainly locate in gastric pit surface and mucus. In the normal control group, no Hp (0/10) is found in the gastric pit and the infection rate is 0%; in the model control group, a large number of blue Hp (11/11) is found in the mucus of the gastric pit and the infection rate is 100%; in the Patchouli Alcohol high dose group, a small number of blue Hp (6/11) is found in the gastric pit and the infection rate is 60%; in the Patchouli Alcohol medium dose group, a small number of blue Hp (7/11) is found in the gastric pit and the infection rate is 63.63%; in the Patchouli Alcohol low dose group, a small number of blue Hp (9/11) is found in the gastric pit and the mucosal surface, and the infection rate is 81.81 %.

In conclusion, the results show that patchouli alcohol has significant *in vitro* and *in vivo* anti*-Helicobacter pylori* effect.

### III. Testing on selective inhibitory effect of patchouli alcohol

1. Testing drug substance: patchouli alcohol, prepared by using the method of preparation according to the disclosure of the Chinese patent application, publication number CN101485647A, with purity greater than 98%. Lansoprazole, obtained from China National Institutes for Food and Drug Control, batch number: 10079-200501, with purity greater than 98%. Clarithromycin, obtained from China National Institutes for Food and Drug Control, batch number: 130356-200403, with purity greater than 98%. Amoxicillin: Product of Zhuhai United Laboratories Inc., batch number: 00800208. Ketoconazole, from Hubei Xing Yinhe Pharmaceutical Company Limited, batch number: 009201.

Strains: *Lactobacillus acidophilus, E. coli, Staphylococcus epidermidis,* yeast, thermophilic Bifidobacterium, *Staphylococcus aureus* (26112), Alpha-Hemolytic *Streptococcus* (32209), Shigella (32013), *Streptococcus pneumoniae* (31001), Catarrhal, *Corynebacterium diphtheriae* (38101), *Escherichia coli* (44113), *Pseudomonas aeruginosa* (10211), *Salmonella Typhi* and *Candida albicans* (98001), Catarrhal is isolated from specimen collected from the throat in the bacterial laboratory, *Candida albicans* is donated by Guangzhou Institute for Food and Drug Control, all other strains are provided by Beijing Institute for Food and Drug Control; *Trichophyton gypseum* (4097), *Trichophyton cerebriforme, Trichophyton tonsurans, Microsporum gypseum, Microsporum lanosum, Epidermophyton floccosum* (previously known as *Trichophyton floccosum*) and *Trichophyton rubrum* are donated by Shanghai Huashan Hospital.

### 3. Culture medium: nutrient broth, 1% dextrose broth, 1% serum broth, Sabouraud agar plate, prepared by conventional preparation method.

### 4. Testing method (In vitro method)

Drug preparation: Use nutrient broth to prepare a series of 14 drug solution in which the content of drug per ml are, 0.1g, 0.05g, 0.025g...1.22×10⁻⁵g respectively and the total volume of each test tube is 1ml, and sterile by steam sterilization. For Streptococcus testing, 1% glucose is added to the sterile drug solution, for *Streptococcus pneumoniae* and *Corynebacterium diphtheria,* 10% inactivated rabbit serum is added, and for *Candida albicans,* the drug solution is prepared by using Sabouraud broth.

Control: for strain control, testing bacteria is added to non-drug culture media; for drug control, no bacteria is added to the drug solution.

For each drug solution at different concentration and for bacteria control, add 1ml of 1:2000 testing bacteria solution (8 hours cultured substance) to each tube, incubate bacteria culture at 37°C for 18 hours and observe the results. For *Candida albicans*, 24 hours cultured substance is used, and incubate bacteria culture at 28°C for 48 hours after bacteria is added to the drug solution and then observe the results. Turbidity is the indicator and visually observe each tube to determine if there is any bacterial growth.

Determine minimum inhibitory concentration (MIC): MIC is the lowest concentration of an antimicrobial that will inhibit the visible growth of a microorganism. The results are shown in Table 3.

**Table 3: In vitro Minimum Inhibitory Concentration MIC of Patchouli Alcohol (mg/ml)**

| Strain | Lansoprazole + amoxicillin + clarithromycin | Ketoconazole | Patchouli Alcohol |
|---|---|---|---|
| *Lactobacillus acidophilus* | < 0.0122 | / | - |
| *E. Coli* | < 0.0122 | / | - |
| *Staphylococcus epidermidis* | < 0.0122 | / | - |
| Yeast | < 0.0122 | / | - |
| Thermophilic Bifidobacterium | < 0.0122 | / | - |
| *Staphylococcus aureus* | < 0.0122 | / | - |
| Alpha- Hemolytic streptococcus | < 0.0122 | / | - |
| Shigella | < 0.0122 | / | - |
| *Streptococcus pneumoniae* | < 0.0122 | / | 0.51 |
| Catarrhal | 6.25 | / | - |
| *Corynebacterium diphtheria* | 3.13 | / | - |
| *Escherichia coli* | 0.0122 | / | - |
| *Salmonella typhi* | < 0.0122 | / | - |
| *Pseudomonas aeruginosa* | 6.25 | / | - |
| *Candida albicans* | / | 12.2 | - |
| *Trichophyton gypseum* (4097) | / | 0.383 | - |
| *Trichophyton cerebriforme* | / | 0.383 | - |
| *Trichophyton tonsurans* | / | 49 | - |
| *Microsporum gypseum* | / | 0.383 | - |
| *Microsporum lanosum* | / | 24.5 | - |
| *Epidermophyton floccosum* | / | 0.383 | - |
| *Trichophyton rubrum* | / | 24.4 | - |

Remarks: Strain Control group for normal growth of bacteria, Drug control group for no bacterial growth, "-" refers to no inhibitory effect, "/"refers to "not tested"

The results in Table 3 shows that patchouli alcohol has a predetermined inhibitory effect on *Streptococcus pneumonia,* but does not show any significant inhibitory effect on the majority of the testing strains (including respiratory, common gastrointestinal probiotics and pathogens).

Conclusion: The above experiment shows that patchouli alcohol can selectively inhibit the growth of *Helicobacter pylori* and does not affect the growth and reproduction of other strains in the above experiment.

### Testing on Toxicity of Patchouli Alcohol in mice

### 1. Testing materials

1.1 Testing drug substance: Patchouli Alcohol, obtained from China Institutes of Food and Drug Control, batch number: 110772-200804, with purity greater than 98%.
1.2 Reagent: Peanut oil, Guangdong Zhangxing Food Trading Company Limited, batch number 090407.
1.3 Testing animal: KM mice, SPF grade, weight 18-22g, equal numbers of male and female, obtained from Animal Laboratory Center of Guangzhou University of Chinese Medicine, Certificate number: SCXK (Yue) 2008-0020.
1.4 Instrument: High precision household electronic scale, Baiyun Zhengli Electronics Factory, model ZL-3.

### 2. Experimental method

2.1 Preparation of drug solution: weight a predetermined quantity of patchouli alcohol and put it into a beaker, add suitable amount of peanut oil into the beaker as solvent, mix while heating until the patchouli alcohol is dissolved. Cool under room condition and then add peanut oil to the predetermined amount to obtain the patchouli alcohol solution.
2.2 Animal grouping: Take 200 mice, weight between 18∼22g, divided by weight and randomly divided into 4 groups, namely: orogastric gavage drug group, orogastric gavage control group (equal volume of peanut oil is used), intraperitoneal injection drug group, Intraperitoneal injection control group (equal volume of peanut oil is used), each group includes 50 mice, equal numbers of male and female, raise for one week before experiment for the mice to adapt to the environment, water fasting for 24 hours before experiment.

### Oral Acute Toxicity

Orogastric gavage patchouli alcohol solution to mice, pre-test to find out the maximum lethal dose and the minimum lethal dose, which are 7.9g/kg and 2.5g/kg respectively. Set the initial dose at 7.90g/kg, at a ratio of 1 : 0.75 divide 5 groups, each group includes 10 mice, equal numbers of male and female, give patchouli alcohol solution to mice once by orogastric gavage according to the dosage at 2.5g/kg, 3.33g/kg, 4.44g/kg, 5.93g/kg, 7.90g/kg for each group. Observe consecutively for 14 days, record toxicity reaction and lethal rate every day, and calculate the medium lethal dose (LD50) by Bliss method.

### 2.4 Intraperitoneal Acute Toxicity

Give patchouli alcohol solution to mice by intraperitoneal injection, pre-test to find out the maximum lethal dose and the minimum lethal dose, which are 6.32g/kg and 2.0g/kg respectively. Set the initial dose at 6.32g/kg, at a ratio of 1: 0.75 divide 5 groups, each group includes 10 mice, equal numbers of male and female, give patchouli alcohol solution to each of the mice once by intraperitoneal injection according to the dosage at 2.0g/kg, 2.67g/kg, 3.56g/kg, 4.70g/kg, 6.32g/kg for each group. Observe consecutively for 14 days, record toxicity reaction and lethal rate every day, and calculate the medium lethal dose (LD50) by Bliss method.

### 3. Experiment Results

Oral Acute Toxicity: after the drug is given for 4 hours, the mice show different levels of unsteady standing, swinging from side to side, convulsion and stiffness phenomena, and even death. Death usually occurs between 6∼12 hours, autopsy is timely carried out for the dead mice, no obvious abnormality is found in major organs, and the surviving animals return to normal after about 24 hours. The results are shown in Table 9.

**Table 9: Death condition of mice which are subject to orogastric gavage of patchouli alcohol peanut oil solution.**

| Group | Dosage(g/kg) | Animal (Count) | Number of Death (count) |
|---|---|---|---|
| 1 | 2.50 | 10 | 0 |
| 2 | 3.33 | 10 | 2 |
| 3 | 4.44 | 10 | 4 |
| 4 | 5.93 | 10 | 7 |
| 5 | 7.90 | 10 | 10 |

The experimental results show that the LD50 of orogastric gavage of Patchouli Alcohol peanut oil solution is 4.693g/kg, where the 95% confidence interval has an upper limit of 5.498g/kg and a lower limit of 4.038g/kg.

Intraperitoneal Acute Toxicity: after the drug is given for 4 hours, the mice show similar behavior as in oral acute toxicity test. Death usually occurs between 6∼12 hours, autopsy is timely carried out for the dead mice, no obvious abnormality is found in major organs, and the surviving animals return to normal after about 24 hours. The results are shown in Table 10.

**Table 10: Death condition of mice which are subject to intraperitoneal injection of patchouli alcohol peanut oil solution.**

| Group | Dosage (g/kg) | Animal (Count) | Death (Count) |
|---|---|---|---|
| 1 | 2.00 | 10 | 1 |
| 2 | 2.67 | 10 | 3 |
| 3 | 3.56 | 10 | 6 |
| 4 | 4.70 | 10 | 9 |
| 5 | 6.32 | 10 | 10 |

The experimental results show that the LD50 of orogastric gavage of patchouli alcohol peanut oil solution is 3.145g/kg, where the 95% confidence interval has an upper limit of 3.675g/kg and a lower limit of 2.663g/kg.

The above acute toxicity tests show that patchouli alcohol is generally safe and non-toxic.

Accordingly, the above experiments show that patchouli alcohol, which is also known as Bai Qiu Li Chun in Chinese pinyin, has a very strong inhibitory effect against *Helicobacter pylori,* is generally safe and non-toxic, and has a very good prospect for development and medical use.

Still further objects and advantages will become apparent from a consideration of the ensuing description.

These and other objectives, features, and advantages of the present invention will become apparent from the following detailed description and the appended claims.

### Detailed Description of the Preferred Embodiment

### Embodiment 1 (dripping pill)

Obtain 30g patchouli alcohol, add 120g PEG4000 as a substrate and suitable amount of liquid paraffin as a cooling agent; prepare by dripping method in which dripping temperature is 80°C, dripping rate is 15 drops per minute, dripping distance is 5cm, length of cooling column is 35cm, interior diameter of dripping mouth is 2.5cm, the temperature of cooling agent is 0°C (ice water bath) and 5000 dripping pills are obtained, each of the dripping pill contains 6mg patchouli alcohol with a net weight of 0.03g. The dripping pills are taken orally to treat stomach illness related to *Helicobacter pylori.* 5∼15 per taking, 1∼3 times per day and 10∼20 days for one treatment course, and 2∼3 consecutive courses can be used for treatment.

### Embodiment 2 (Injection)

Obtain 150mg patchouli alcohol, add 9g sodium chloride, add water to 1000ml, adjust pH to 3.0-5.5 by using 1 mol/ml sodium hydroxide, filter and seal the filtrate in a 2, 5 or 10ml ampoule, and sterile at 100°C for 30 minutes to obtain the injection. The drug is given by injection to treat stomach illness related to *Helicobacter pylori* and can be given by intravenous or intramuscular injection. 1∼50ml per injection, 1∼3 times per day and 10∼20 days for one treatment course, and 2∼3 consecutive courses can be used for treatment.

### Embodiment 3 (soft capsule)

Weigh 30g patchouli alcohol, mix with 60g edible corn oil, mix thoroughly to obtain the patchouli alcohol content of the soft capsule; weigh and obtain suitable amount of gelatin, glycerin and water (where the mass ratio of gelatin: glycerol: water = 1: 0.5: 1) and prepare the capsule shell liquid; place the patchouli alcohol content and the capsule shell liquid into the automatic capsule filling machine by rotating and pressing method to prepare 500 counts of soft capsules, each of which contains 60mg patchouli alcohol with a net weight of 0.18g. A suitable amount of liquid paraffin is used as lubricant. 1∼3 capsule per taking, 3 times per day and 10∼20 days for one treatment course, and 2∼3 consecutive courses can be used for treatment.

### Embodiment 4 (Tablet)

Obtain 40g patchouli alcohol, add 480g lactose and 1100g starch, then mix uniformly, use 300g 7% starch paste as a binding agent and prepare the tablet by wet granulation, oven dry, mix with 16g magnesium stearate and compress to prepare 10000 tablets, each of which contains 4mg Patchouli Alcohol with a net weight of 0.17g. Taken orally to treat stomach illness related to *Helicobacter pylori.* 3∼9 tablets per taking, 3 times per day and 10∼20 days for one treatment course, and 2∼3 consecutive courses can be used for treatment.

### Embodiment 5 (soft capsule)

Weigh 70g patchouli alcohol, mix with 30g edible corn oil, increase temperature to 60°C to dissolve, mix thoroughly to obtain the patchouli alcohol content of the soft capsule; weigh and obtain suitable amount of gelatin, glycerin and water (where the mass ratio of gelatin: glycerol: water = 1: 0.5: 1) and prepare the capsule shell liquid; place the patchouli alcohol content and the capsule shell liquid into the automatic capsule filling machine by rotating and pressing method to prepare 500 soft capsules, each of which contains 140mg Patchouli Alcohol with a net weight of 0.2g. A suitable amount of liquid paraffin is used as lubricant. 1∼2 capsule per taking, 2 times per day, and 10∼20 days for one treatment course, and 2∼3 consecutive courses can be used for treatment.

### Embodiment 6 (Tablet)

Obtain 8g patchouli alcohol, add 480g lactose and 1150g starch, then mix uniformly, use 350g 7% starch paste as a binding agent and prepare the tablet by wet granulation, oven dry, mix with 16g magnesium stearate and compress to prepare 10000 tablets, each of which contains 0.8mg patchouli alcohol with a net weight of 0.17g. Taken orally to treat stomach illness related to *Helicobacter pylori.* 3∼9 tablets per taking, 3 times per day, and 10∼20 days for one treatment course, and 2∼3 consecutive courses can be used for treatment.

## Claims

1. Patchouli alcohol for use in the treatment of Helicobacter pylori infection.

2. Patchouli alcohol for use according to claim 1, **characterized in that** a pharmaceutical preparation is used comprising the patchouli alcohol.

3. Patchouli alcohol for use according to claim 2, **characterized in that** the patchouli alcohol is the only compound within the pharmaceutical preparation that is active against the Helicobacter pylori infection.

4. Patchouli alcohol for use according to claim 2, **characterized in that** the content of patchouli alcohol is 0.5 ∼ 70% by percentage weight and wherein the pharmaceutical preparation comprises pharmaceutically acceptable additives.

5. Patchouli alcohol for use according to claim 4, **characterized in that** the patchouli alcohol is administered by injection or orally.

## Patentansprüche

1. Patschulialkohol zur Verwendung bei der Behandlung einer Helicobacter-Pylori-Infektion.

2. Patschulialkohol zur Verwendung nach Anspruch 1, **dadurch gekennzeichnet, dass** ein den Patschulialkohol umfassendes pharmazeutisches Präparat verwendet wird.

3. Patschulialkohol zur Verwendung nach Anspruch 2, **dadurch gekennzeichnet, dass** der Patschulialkohol die einzige gegen die Helicobacter-Pylori-Infektion wirksame Zusammensetzung innerhalb des pharmazeutischen Präparats ist.

4. Patschulialkohol zur Verwendung nach Anspruch 2, **dadurch gekennzeichnet, dass** der Gehalt an Patschulialkohol 0,5 - 70 Gewichtsprozent beträgt, und wobei das pharmazeutische Präparat pharmazeutisch annehmbare Zusatzstoffe umfasst.

5. Patschulialkohol zur Verwendung nach Anspruch 4, **dadurch gekennzeichnet, dass** der Patschulialkohol durch Injektion oder oral verabreicht wird.

## Revendications

1. Alcool de patchouli utilisé pour le traitement d'une infection par Helicobacter pylori.

2. Alcool de patchouli destiné à être utilisé selon la revendication 1, **caractérisé en ce qu'**une préparation pharmaceutique contenant l'alcool de patchouli est utilisée.

3. Alcool de patchouli destiné à être utilisé selon la revendication 2, **caractérisé en ce que** ledit alcool de patchouli est l'unique composé dans la préparation pharmaceutique qui est actif contre l'infection par Helicobacter pylori.

4. Alcool de patchouli destiné à être utilisé selon la revendication 2, **caractérisé en ce que** la teneur en alcool de patchouli est comprise entre 0,5 et 70 % en poids, et où la préparation comprend des additifs pharmaceutiquement acceptables.

5. Alcool de patchouli destiné à être utilisé selon la revendication 4, **caractérisé en ce que** l'alcool de patchouli est administré par injection ou par voie orale.
